# EUROPÄISCHE PATENTANMELDUNG

(11) **EP 0 998 954 A2**
(43) Veröffentlichungstag der Anmeldung: **10.05.2000**
(21) Anmeldenummer: 99121375.2
(22) Anmeldetag: 27.10.1999
(51) Int. Cl.: A61M 16/14, A61M 11/06

(54) **Parfümierung von Sauerstoff**

(30) Priorität: 07.11.1998 DE 19851380
(71) Anmelder: MESSER GRIESHEIM GMBH, 60547 Frankfurt (DE)
(72) Erfinder: Kunkel, Randolf, 47055 Duisburg (DE); Schucht, Fridtjof, Dr., 47803 Krefeld (DE); Biallas, Ralf, 47228 Duisburg (DE); Dammers, Irmhild, 47199 Duisburg (DE)

(57) **Zusammenfassung**

Die Vorrichtung zur Herstellung parfümierter sauerstoffhaltiger Gase enthält eine Gasquelle 1 mit einem Betätigungsventil 2, ein Vorratsgefäß 4 für einen Duftstoff 7 und eine Kapillare 5 zur Abgabe des Duftstoffes 7 in eine Leitung 3 mit dem strömenden sauerstoffhaltigen Gas. Die Vorrichtung wird in einem Verfahren zur Herstellung parfümierter sauerstoffhaltiger Gase eingesetzt.

## Beschreibung

Die Erfindung betrifft eine Vorrichtung und ein Verfahren zur Parfümierung von sauerstoffhaltigen Gasen wie Sauerstoff, synthetische oder natürliche Luft.

Patienten mit chronischen Atembeschwerden (z. B. Asthma und COPD (chronisch obstruktive Atemwegserkrankung / Chronic Obstructive Pulmonary Disease)) werden durch einen in der Regel transportablen Sauerstoffspender in der Sauerstoffversorgung des Körpers unterstützt. Solche sogenannten spontanatmenden Patienten erhalten so zum Beispiel eine zusätzliche Sauerstoffspende (LOT = Langzeitsauerstofftherapie) oder eine Atmungsunterstützung (per CPAP = continuous posit. airways pressure).

Ein Gerät zur Zufuhr von Atemgas oder Sauerstoff zu einem Patienten wird in DE 43 09 923 A1 beschrieben.

Der Erfindung liegt die Aufgabe zugrunde, Patienten mit parfümierten sauerstoffhaltigen Gasen zu versorgen, ohne daß eine vorzeitige Zersetzung des Duftstoffes durch die sauerstoffhaltigen Gase auftritt.

Gelöst wurde die Aufgabe durch ein Verfahren zur Parfümierung sauerstoffhaltiger Gase mit einem Duftstoff, der durch eine Kapillare dem Gas beigefügt wird.

Sauerstoffhaltige Gase sind z. B. Sauerstoff, synthetische Luft und natürliche Luft.

Das sauerstoffhaltige Gas wird aus einer Gasquelle bereitgestellt. Als Gasquelle dienen in der Regel Druckgasbehälter mit dem komprimierten Gas oder Behälter mit kälteverflüssigten Gasen.

Der Sauerstoff ist vorzugsweise reiner Sauerstoff. Der Sauerstoff kann auch andere Gase enthalten. Das sauerstoffhaltige Gas enthält im allgemeinen mindestens 20 Volumenprozent Sauerstoff. Bevorzugt werden sauerstoffhaltige Gase mit mindestens 50 Volumenprozent Sauerstoff und besonders bevorzugt mit mindestens 80 Volumenprozent Sauerstoff. Reines Sauerstoffgas fällt unter den Begriff "sauerstoffhaltiges Gas".

Duftstoffe sind Riechstoffe, die beim Menschen ein angenehmes Geruchsempfinden auslösen. Duftstoffe sind vorzugsweise gasförmig oder leicht verdampfbar. Geeignete Duftstoffe sind beispielsweise etherische Öle.

Zur Parfümierung oder Aromatisierung wird dem sauerstoffhaltigen Gas ein Duftstoff zugesetzt. Duftstoffe oder Aromen sind in der Regel gasförmige oder flüchtige flüssige oder flüchtige feste Verbindungen, In der Regel werden Duftstoffe als Mischungen verschiedenster Duftstoffe eingesetzt (z. B. als Duftstoff-Konzentrat). Duftstoffe sind beispielsweise etherische Öle, die im allgemeinen Terpene enthalten. Die Duftstoffe dienen bevorzugt zur Erzeugung eines wohlriechenden sauerstoffhaltigen Gases. Die Duftstoffe können außer einem angenehmen Geruch in der Anwendungskonzentration auch eine physiologische oder andere vorteilhafte Wirkung aufweisen. Beispielsweise wirkt Menthol desodorierend. Insbesondere die Behandlung der Atemwege mit Menthol ist vorteilhaft.

Duftstoffe enthalten beispielsweise natürliche oder naturidentische Aromastoffe wie Vanillin, Biacetyl, Eucolaptyl.

Duftstoffe sind im allgemeinen natürliche, halbsynthetische oder vollsynthetische Stoffe. Natürliche Duftstoffe sind z. B. Pfefferminzöl, Lavendelöl, Rosenöl, Patchouliöl, Nelkenöl. Natürliche Duftstoffe werden bevorzugt durch Extraktion in überkritischen Gasen, insbesondere Kohlendioxid, hergestellt und in diesen Gasen eingesetzt.

Das sauerstoffhaltige Gas kann physiologisch wirksame Substanzen, wie Terpene oder andere wirksame Bestandteile wie etherische Öle, deren therapeutische oder anregende, positive Wirkung auf den menschlichen Organismus aus der Medizin bekannt ist, enthalten und damit in einfacher Weise in der Therapie oder der Selbstmedikation inhalativ appliziert werden.

Der Duftstoff ist vorzugsweise ein Gemisch und enthält Ester, Aldehyde, Ketone, Alkohole, Lactone, Terpen-Kohlenwasserstoffe und/oder etherische Öle. Alkohole sind beispielsweise Menthol und Phenylethylalkohol. Besonders bevorzugte Duftstoffe enthalten Ester, in der Regel 30 bis 70 Vol.-% (bezogen auf die Gesamtmenge des Duftstoffes), insbesondere 30 bis 60 Vol.-%. Diese Duftstoffe und Duftstoffgemische sind in der Regel bei Raumtemperatur flüssig.

Vorzugsweise werden zur Parfümierung von sauerstoffhaltigem Gas oxidationsstabile Verbindungen als Duftstoffe eingesetzt, wie Ester (z. B. Essigsäureethylester, Menthylacetat, organische Ester), Ketone (z. B. Menthon, Carvon) oder Ether wie Diphenylether oder t-Butylmethylether.

Auch natürliche oder natürlich vorkommende Duftstoffe, die oxidationsempfindliche funktionelle Gruppen wie Hydroxylgruppen enthalten, können durch geeignete Derivatisierung (Einführung von Schutzgruppen; insbesondere Veresterung) in stabilere Gruppen überführt werden. Beispielsweise werden Hydroxylgruppen verestert.

Vorzugsweise enthalten die eingesetzten Duftstoffe keine oxidationsempfindlichen Lösemittel wie Ethanol.

Der Lösemittelgehalt der Duftstoffe ist vorzugsweise niedrig, z. B. 0,1 bis 1 Vol.-% oder niedriger.

Duftstoffkonzentrate sind z. B. die verschiedensten Parfümöle.

Der Zusatz und die Dosierung von Duftstoffen in das sauerstoffhaltige Gas erfolgen mittels Diffusion der Duftstoffe aus einem Vorratsgefäß durch eine Kapillare in das vorbeiströmende sauerstoffhaltige Gas.

Durch Diffusion der Duftstoffformulierung durch eine ausreichend kleine Kapillaröffnung läßt sich bei Raumtemperatur ein hinreichender Stoffstrom einstellen, der über eine einfache Anbindung an die das sauerstoffhaltige Gas führende Leitung zu einer Parfümierung des sauerstoffhaltigen Gases führt. Die Diffusion erfolgt aus einem Vorratsgefäß (z. B. 5 bis 25 ml Volumen) bei 20 bis 30 °C durch eine z. B. 2 bis 10 cm lange Kapillare mit in der Regel 0,1 bis 1 mm lichter Weite. Es stellen sich unter diesen Bedingungen in der Regel Konzentrationen von 0,1 bis 50 mg/m³ Duftstoff im sauerstoffhaltigen Gas ein. Durch die enge Kapillaröffnung wird der Stoffstrom auf ein gewünschtes Minimum beschränkt, die Kapillaröffnung verhindert die Entstehung brennbarer Atmosphären und verhindert sicher eine Rückzündung der organischen Duftstoffe im Vorratsgefäß der Duftstoffe. Überraschenderweise gelingt eine gleichmäßige Duftstoffabgabe in das sauerstoffhaltige Gas ohne eine merkliche Entmischung der Duftstoffkomponenten im Vorratsgefäß bei Einsatz von Duftstoffgemischen.

Der Massenstrom (m) des Duftsstoffes ist direkt proportional dem Gesamtdruck P des austretenden Gasstromes (Duftstoff, in der Regel 1-1,5 bar), der relativen Molmasse des Duftstoffes und dem inneren Kapillarquerschnitt (A) und umgekehrt proportional der Temperatur (T) und der Länge (I) der Kapillare.
Es gilt: m = P * M * A * D / (R * T * I) * In (P / (P - P₀)), wobei P₀= Dampfdruck der Substanz, R = Gaskonstante, D = Diffusionskoeffizient (ca. 2,4 * 10⁻³ m²/s), In = natürlicher Logarithmus ist.
Bei einem Dampfdruck von ca. 0,1 bis 10 mbar, T = 300 K, einer Kapillarlänge I= 5 cm und einer lichten Weite A = 0,35 mm ergibt sich bei einer angenommenen Molmasse von 150 bis 300 Atommasseneinheiten ein möglicher Massenstrom m von 0,05 bis 25 µg/min. Mit einem Sauerstofffluß von ca. 5 Liter/min. ergibt sich daraus eine Konzentration von 10 bis 5000 µg/m³, bzw. 0,1 bis 50 ppm (bei Molmasse 225).

Die Kapillare besteht in der Regel aus Glas oder Metall, vorzugsweise Edelstahl.

Die Dosierung von Duftstoff in das sauerstoffhaltige Gas kann durch gesteuerte Temperatureinwirkung geregelt werden. Beispielsweise kann die Temperatur der Kapillare und/oder des Vorratsgefäßes durch eine Heizwendel (Widerstandsheizung) eingestellt werden.

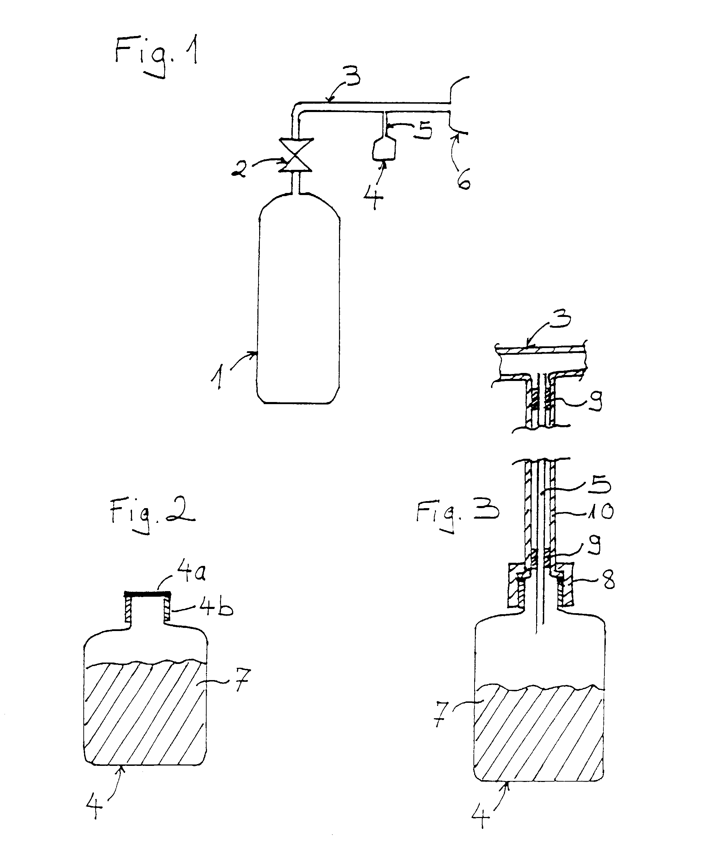

Fig. 1 zeigt ein Schema einer Vorrichtung zur Parfümierung von sauerstoffhaltigen Gasen, insbesondere reinem Sauerstoff aus einem Druckbehälter. Die als Beispiel gezeigte Vorrichtung besteht im wesentlichen aus einer Gasquelle 1 (z. B. Druckdose als Druckgasbehälter mit Sauerstoff) mit einem Betätigungsventil 2, einer Gaseleitung 3, einem Vorratsgefäß 4 für den Duftstoff mit angeschlossener Kapillare 5 und einer Gesichtsmaske 6.

Fig. 2 zeigt im Schema eines geeignetes Vorratsgefäß 4 für den Duftstoff 7. Das Vorratsgefäß 4 ist vorzugsweise ein Behälter mit einem Hals, der ein Gewinde 4b trägt. Das Vorratsgefäß 4 ist vorteilhaft mit einer Metallfolie 4a verschlossen, die bei Anbringung der Kapillare 5 am Vorratsgefäß von der Kapillare durchstoßen wird. Das Vorratsgefäß 4 ist vorteilhaft ein Glasgefäß oder eine Glasampulle mit einem Fassungsvermögen im Bereich von 5 bis 25 ml, vorzugsweise 10 bis 20ml.

Fig. 3 zeigt ein Beispiel für eine bevorzugte Ausführungsform der Vorrichtung gemäß der Erfindung, wobei nur der Bereich Gasleitung 3/ Kapillare 5 Vorratsgefäß 4 abgebildet ist. Gaseleitung 3 und Rohr 10 sind in dem gezeigten Beispiel ein Edelstahlrohr mit 6 mm Innendurchmesser. Die Kapillare 5 ist mittels der abdichtenden Lötverbindungen 9 im Rohr 10 fixiert. Die Kapillare ist z. B. ein Edelstahlrohr mit 1/8 Zoll (ca. 3 mm) Außendurchmesser und 0,1 bis 1mm Innendurchmesser. Vorteilhaft ist das dem Vorratsgefäß 4 zugewandte Ende der Kapillare 5 abgeschrägt. Als Kapillare 5 kann z. B. auch ein Teil einer Injektionsnadel verwendet werden. Das Rohr 10 mit der Kapillare 5 wird z. B. mit einer Überwurfmutter 8 am Vorratsgefäß 4 befestigt.

### Ausführungsbeispiel

Der Vorratsbehälter für den Duftstoff ist z. B. derart ausgeführt, daß auf einem Metallgefäß von etwa 20 ml Inhalt ein Gewinde für eine 1/8 Zoll-Verschraubung angebracht ist. Durch die 1/8 Zoll-Verschraubungsüberwurfmutter führt eine eingelötete Kapillare einer bestimmten Länge mit einem Außendurchmesser von 2 bis 3,2 mm und einem Innendurchmesser von 0,1 bis 1mm, vorzugsweise 0,2 bis 0,5 mm. Nach Abnahme der Verschraubungsüberwurfmutter läßt sich das Gefäß mit einer Duftstoffformulierung befüllen. Die Kapillare ist in ihrem oberen Ende in ein ¼ Zoll-Rohrstück eingelötet und kann somit mittels einer 6 mm (1/4 Zoll)-Verschraubung an die Gaseleitung für den Sauerstoffstrom angeschlossen werden. Das Vorratsbehälter-Kapillarensystem kann ausgangsseitig mit einem Originalitätsverschluß, z. B. eine dünne Metallmembran oder eine selbstschließende Abdichtung, z. B. mit Feder und Kugel oder ein anderes Absperrorgan versehen sein. Das Absperrorgan kann für einen einmaligen oder mehrmaligem Gebrauch ausgelegt sein. Beispielsweise stellt das Durchstoßen einer Metallmembran einen einmaligen Gebrauch dar. Das Absperrorgan kann für mehrmalige Funktion ventilartig aufgebaut sein, z. B. ein Ventil, das bei manueller Betätigung geöffnet wird (z. B. Stößel, der bei Einschrauben oder Druck das Ventil öffnet).

### Bezugszeichenliste

- 1: Quelle für sauerstoffhaltiges Gas (z. B. Druckgasbehälter)
- 2: Betätigungsventil
- 3: Gaseleitung
- 4: Vorratsgefäß für Duftstoff
- 4a: Metallfolie
- 4b: Gewinde
- 5: Kapillare
- 6: Atemmaske oder Gesichtsmaske
- 7: Duftstoff
- 8: Überwurfmutter
- 9: Lötverbindung
- 10: Rohr (Schutzrohr; Rohr zur Montage der Kapillare)

## Patentansprüche

1. Vorrichtung zur Herstellung parfümierter sauerstoffhaltiger Gase, enthaltend eine Gasquelle 1 mit einem Betätigungsventil 2, ein Vorratsgefäß 4 für einen Duftstoff 7 und eine Kapillare 5 zur Abgabe des Duftstoffes 7 in eine Leitung 3 mit dem strömenden sauerstoffhaltigen Gas.

2. Vorrichtung nach nach Anspruch 1, dadurch gekennzeichnet, daß ein Druckgasbehälter mit komprimiertem sauerstoffhaltigem Gas oder ein Behälter mit kälteverflüssigtem sauerstoffhaltigen Gas als Gasquelle 1 eingesetzt wird.

3. Vorrichtung nach Anspruch 1, dadurch gekennzeichnet, daß eine Kapillare 5 aus Glas oder Metall mit einer Länge von 2 bis 10 cm und einer lichten Weite von 0,1 bis 1 mm eingesetzt wird.

4. Vorrichtung nach einem der Ansprüche 1 bis 3, dadurch gekennzeichnet, daß das System Kapillare 5/Vorratsgefäß 4 ein Absperrorgan enthält.

5. Vorrichtung nach Anspruch 4, dadurch gekennzeichnet, daß das Absperrorgan je nach Bedarf geöffnet und geschlossen werden kann.

6. Vorrichtung nach Anspruch 4 oder 5, dadurch gekennzeichnet, daß das Absperrorgan bei Öffnung des Betätigungsventiles 2 der Gasquelle 1 geöffnet ist.

7. Verfahren zur Parfümierung sauerstoffhaltiger Gase mit einem Duftstoff 7, der durch eine Kapillare 5 dem sauerstoffhaltigen Gas zugeführt wird.

8. Verfahren nach Anspruch 7, dadurch gekennzeichnet, daß eine Kapillare aus Glas oder Metall mit einer Länge von 2 bis 10 cm und einer lichten Weite von 0,1 bis 1 mm eingesetzt wird.

9. Verfahren nach Anspruch 7 oder 8, dadurch gekennzeichnet, daß der Duftstoff 7 dem sauerstoffhaltigen Gas temperaturgeregelt zugeführt wird.
